# EUROPEAN PATENT SPECIFICATION

(11) **EP 2 750 600 B1**
(45) Date of publication and mention of the grant of the patent: **12.04.2017**
(21) Application number: 12787079.8
(22) Date of filing: 13.09.2012
(51) Int. Cl.: A61B 5/083, A61J 3/07, A61K 9/48, A61K 47/08, A61K 47/10, A61K 47/38, A61K 49/00, A61B 5/08

(54) **FILL FORMULATIONS AND CAPSULES AND METHOD OF USE TO AVOID MIGRATION OF FILL INTO OR THROUGH THE SHELL**
FÜLLFORMULIERUNGEN UND KAPSELN SOWIE VERFAHREN ZUR VERHINDERUNG DER MIGRATION DER FÜLLUNG IN ODER DURCH DIE SCHALE
GÉLULES ET FORMULATIONS DE REMPLISSAGE ET PROCÉDÉ D'UTILISATION DESTINÉ À ÉVITER LA MIGRATION DU REMPLISSAGE DANS OU À TRAVERS L'ENVELOPPE

(30) Priority: 11.01.2012 US 201261585459 P
(43) Date of publication of application: 09.07.2014
(73) Proprietor: Capsugel Belgium NV, 2880 Bornem (BE)
(72) Inventor: VERTOMMEN, Jan, B-1851 Humbeek (BE); DELMARRE, David, F-67400 Illkirch (FR); MARTINA, Marie-sophie, F-6700 Strasbourg (FR)
(74) Representative: De Clercq & Partners
(86) International application number: PCT/IB2012/002211
(87) International publication number: WO 2013/038271

(56) References cited:
- US-A- 5 665 386
- US-A1- 2003 194 431
- KRISHNAIAH Y S R ET AL: "Enhanced percutaneous permeability of nicardipine hydrochloride by carvone across the rat abdominal skin", DRUG DEVELOPMENT AND INDUSTRIAL PHARMACY, NEW YORK, NY, US, vol. 29, no. 2, 1 January 2003 (2003-01-01), pages 191-202, XP008159230, ISSN: 0363-9045, DOI: 10.1081/DDC-120016727
- MARTIN KUENTZ ET AL: "Determination of the optimal amount of water in liquid-fill masses for hard gelatin capsules by means of texture analysis and experimental design", INTERNATIONAL JOURNAL OF PHARMACEUTICS, vol. 236, no. 1-2, 1 April 2002 (2002-04-01), pages 145-152, XP55049064, ISSN: 0378-5173, DOI: 10.1016/S0378-5173(02)00022-4

## Description

In one aspect, the present disclosure relates to capsules as defined in claim 1.

In one aspect, the present disclosure relates to fill formulations for capsules and capsules comprising said formulations. In one aspect, the present disclosure also relates to the use of the fill formulations and capsules in a method that avoids migration of the fill formulation contents into the capsule shell or through the capsule shell. The formulations and capsules according to the present disclosure have many uses, both in pharmaceutical and non-pharmaceutical fields. Non-limiting examples of suitable uses are product traceability, such as commercial or clinical trial patient compliance and product performance methods, and product security and integrity, such as anti-counterfeiting methods, tamper evidence methods, clinical trials, disease management, and overall increased capsule integrity and stability.

### Background

The migration of fill contents into or through the capsule shell is a longstanding problem in the capsule art and limits the type of chemical compounds that can be filled, delivered or administered in capsules. This is an issue both for components of the capsule fill having an integral part in the final use of the capsule, such as an active pharmaceutical product (API) when the fill is a dosage form, and inert components within the capsule, such as low molecule weight excipients that provide a diluent or vehicle function to dosage forms. One class of particularly difficult fill ingredients are volatile or semi-volatile compounds such as taggants. This fill problem also impacts non-pharmaceutical uses of capsules as a delivery means and limits the scope of applicability of this type of packaging to the full variety of chemical contents. Whether or not a chemical will migrate into or through a capsule shell from a fill depends upon both its own structure and the particular chemical formula of the capsule shell. These issues have been addressed before, as summarized in Table 3.

**Table 1. Selection of Patent Related to Improving Capsule Integrity**

| **U.S. Patent No.** | **Fill Component at issue** | **Proposed Solution** |
|---|---|---|
| 7,011,846 | 2-pyrrolidone and related solubilizers | Fatty acid stabilizer in fill |
| 5,376,381 | Ethanol (at least 6%) | Phospholipids in fill |
| 5,037,698 | Hygroscopic or deliquescent component | Water equilibrium and propylene glycol in fill |
| 4,780,316 | Polyethylene glycol | Sorbitol, sorbitans, in shell and glycerin, sorbitol or propylene glycol in fill |
| 4,777,048 | Dust-producing, solid API | Polyalkylene glycol lubricant in fill |
| 2,780,355 | Hygroscopic solids or water soluble materials with high | Polyethylene sorbitan monooleate in fill |
| | | |
| | degree of dissociation | |

Moreover, Martin Kuentz ET AL: "Determination of the optimal amount of water in liquid-filled masses for hard gelatin capsules by means of texture analysis and experimental design", International Journal of Pharmaceutics, vol. 236, no. 1-2, 2002-04-01, pages 145-152, describes a study using texture analysis as a nondestructive test for hard gelatin capsules filled with liquid formulations to investigate mechanical changes upon storage. The individualized solutions proposed above are costly and unpredictable, and fail short of providing a solution to the migration issue. Thus, there remains a need in the art for broad-based fill formulations and administration means, such as capsules, that provide improved storage and delivery characteristics for fill contents that tend to migrate into or through the shell of the capsule, thereby compromising the shell's integrity, in certain aspects, the present disclosure provides fill formulations and capsules that preserve the integrity of the shell and minimize degradation. The present and methods utilizing the capsules.

### Summary

In one aspect, the present disclosure relates to a capsule as defined in Claim 1.

In another aspect, the present disclosure also relates to a capsule comprising the fill formulations described above, in one embodiment the present disclosure is directed to a capsule filled with a fill formulation comprising a composition comprising at least one polymer and at least one fill component, wherein the at least one fill component, if not mixed with the polymer, will migrate into or through the capsule shell. The methods according to the present disclosure can be used with hard capsules and soft capsules. The methods according to the present disclosure can be used with capsules that are sealed or banded. In one embodiment of the present disclosure the capsule has its primary component by weight selected from the group consisting of cellulose ether, gelatin, pullulan and mixtures thereof. The primary component of the capsule composition by weight can consists of gelatin. If the primary component of the capsule by weight is cellulose ether it can consists of hydroxypropyl methylcellulose (HPMC).

An additional aspect of the present disclosure is a capsule comprising an inner and an outer capsule, wherein the inner capsule comprises a fill formulation according to the present disclosure and the outer capsule comprises the inner capsule and a further component selected from, for example, therapeutic drugs, pharmaceutical dosage forms, and placebo formulations. The present disclosure also contemplates a capsule comprising an inner and an outer capsule wherein the inner capsule comprises a component selected from therapeutic drugs, pharmaceutical dosage forms, and a placebo formulations, and the outer capsule comprises the inner capsule and fill formulation according to the present disclosure. Where two or more capsules are present, they can have the same or different compositions. The inner capsule may be fixed to the outer capsule to avoid separation between inner and outer capsule or a physical separator may be added to keep the inner capsule within a defined space, within the outer capsule.

Another aspect of the present disclosure is a method of avoiding, preventing and/or retarding migration of a fill component into or through a capsule shell comprising mixing said fill component with a polymer to form a gel- like, matrix prior to its loading into the capsule shell. The polymer is hydroxypropyl cellulose (HPC). The fill comprises 80% -99.9% w/w of 2-butanol, 2-pentanone, and L-carvone and mixtures thereof.

Further aspects, features and advantages of the present innovation will be better appreciated upon a reading of the detailed description.

### BRIEF DESCRIPTION OF THE DRAWINGS

Figure 1: LICAPS^{®} Liquid Capsules by Capsugel, inc., size 000-4,
Figure 2: CONI-SNAP^{®} Hard Gelatin Capsules by Capsugel, inc., size 000 to 0.
Figure 3: DBCAPS^{®} two-piece gelatin or hydroxypropyl methyl cellulose Capsules by Capsugel, Inc., size AAA to E.
Figure 4: is a graphical representation of weight of handfilled 50 microliter 2-butanol in capsules #3 (not sealed) stored at 40°C in a ventilated oven versus time.
Figure 5 depicts the deformation of a LICAPS capsule which contained 225 µL following 3 months storage at 40°C/75%RH.
Figure 6 graphically illustrates that evolution of the weight of manually filled and non sealed capsules (50 µL) in function of days of storage at 40° C. Error bars shown in the Figure represent the minimum and maximum values for the six capsules tested.
Figure 7 graphically illustrates the evolution of the weight of manually filled and non sealed capsules (225 µL) in function of days of storage at 40°C. Error bars shown in the Figure represent the minimum and maximum values for the six capsules tested.
Figure 8 graphically illustrates the evolution of the weight of manually filled and CFS^{®} sealed capsules (50µL) in function of days of storage at 40°C. Error bars shown in the Figure represent the minimum and maximum values for the six capsules tested.
Figure 9 graphically illustrates the evolution of the weight of filled and sealed capsules (225µL) in function of days of storage at 40°C. Error bars shown in the Figure represent the minimum and maximum values for the six capsules tested.
Figure 10 is a graphical illustration of viscosity at 21 °C in function of the percentage of HPC added.

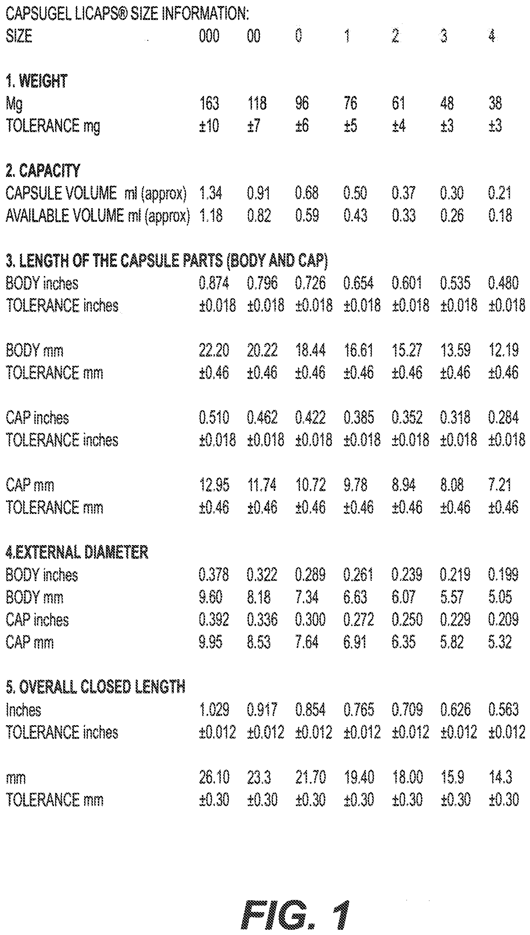

Figure 1 1 is a graphical representation of weight of handfilled 50 microliter 2-butanol + 0.75% HPC in capsules #3 (not sealed) stored at 40°C in a ventilated oven versus time.

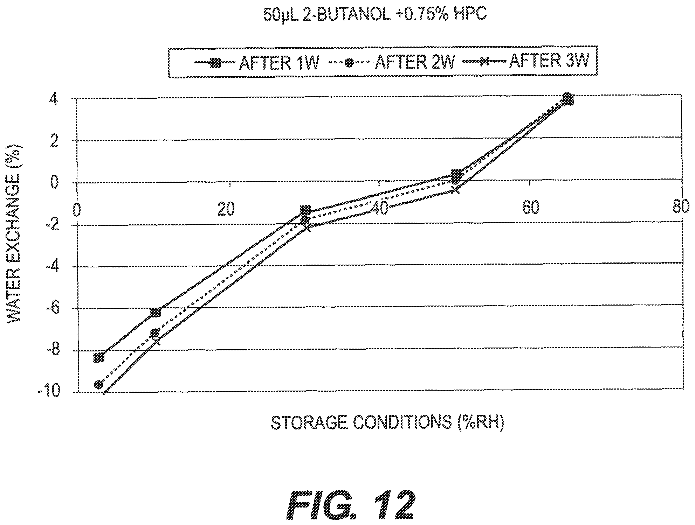

Figure 12 is a graphical representation of hygroscopictty test results for transparent size 3 LICAPS^{®} filled with 50 µL of 2-butanol + 0.75% (w/v) HPC.

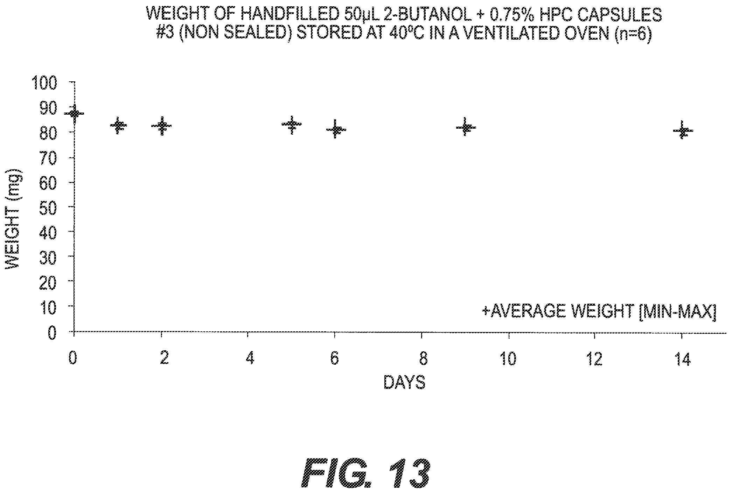

Figure 13 graphically illustratates the evolution of the weight of manually filled and non sealed capsules containing 2-butanol (50µL) + 0.75% HPC in function of days of storage at 40°C. Error bars shown in the Figure represent the minimum and maximum values for the six capsules tested.

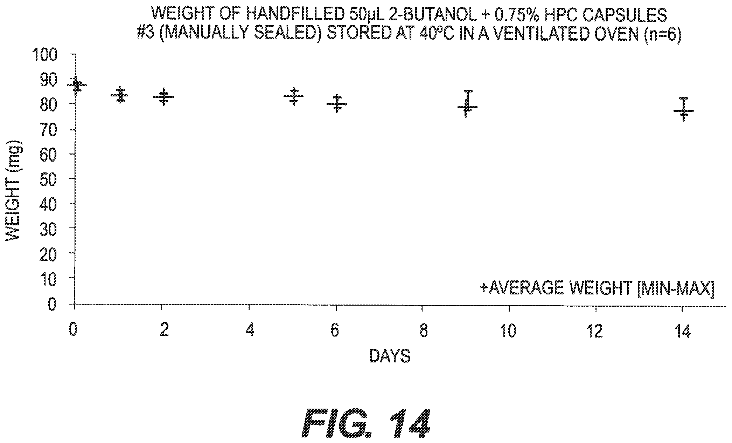

Figure 14 graphically illustrates the evolution of the weight of manually filled and sealed capsules containing 2-butanol (50µL) + 0.75% HPC in function of days of storage at 40°C. Error bars shown in the Figure represent the minimum and maximum values for the six capsules tested.

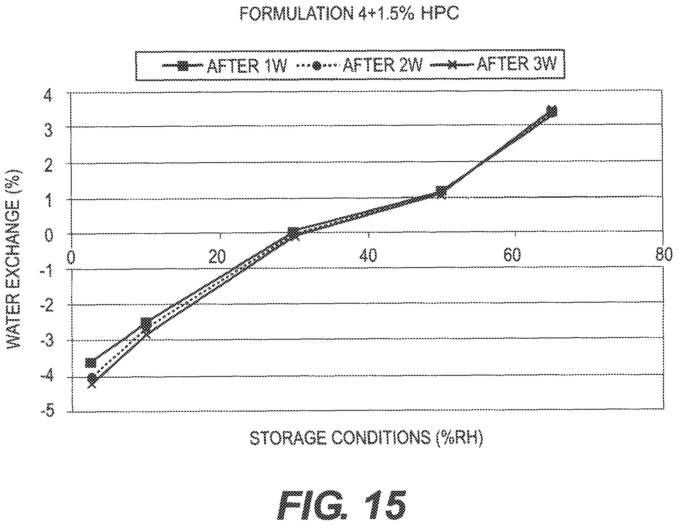

Figure 15 is a graphical illustration of hygroscopicity test results for die transparent size 3 LICAPS^{®} capsules filled with formulation 4 4. 1.5% HPC.

### DETAILED DESCRIPTION OF THE EMBODIMENTS

As used in the present disclosure, the following words, phrases, and symbols are generally intended to have the meanings as set forth below, except to the extent that the context in which they are used indicates otherwise.

As used herein, "optional" or "optionally" means that the subsequently described even or circumstance may or may not occur, and that the description includes instances where the event or circumstance occurs and instances in which it does not.

As used herein, "w/w %" means by weight as a percentage of the total weight.

As used herein, W/v%" means by weight as a percentage of the total volume,

The term "about" is intended to mean approximately, in the region of, roughly, or around. When the term "about" is used in conjunction with a numerical range, it modifies that range by extending the boundaries above and below the numerical values set forth. Unless otherwise indicated, it should be understood that the numerical parameters set forth in the following specification and attached claims are approximations. At the very least, and not as an attempt to limit the application of the doctrine of equivalents to the scope of the claims, numerical parameters should be read in Sight of the number of reported significant digits and the application of ordinary rounding techniques.

To fill a capsule, a solid dispersion or solution of fill components is prepared. If in solution, this often involves a lipid or oil-based vehicle. As described above, certain fill components which would offer advantages in capsule form have been difficult to encapsulate. In some instances these components are insoluble in the usual oil-based vehicles and therefore migrate into the shell causing it to disintegrate, deteriorate or penetrate, the capsule shell. Examples of such substances include hygroscopic solids which have hydrolytic action on the capsule shell, and include fill components such as chloral hydrate or choline chloride. Water-soluble materials having a high degree of dissociation are a further example of such destructive fill components and include salts of strong acids and bases, such as sodium chloride. Other fill components that have a tendency to migrate through the capsule shell are low molecular weight alcohols including ethanol, iso-propanol, and pentanois. These fill components share the characteristics of being volatile, semi-volatile or highly volatile.

Generally, "volatile" is defined as having an increased tendency to undergo the phase transition of from solid or liquid phase to gas phase. Such substances tend to have high vapor pressure and low boiling points.

The present disclosure encompasses a fill formulation that comprises one or more fill components that migrate into a capsule shell and a polymer, wherein the polymer has been mixed with the fill component to form a gel-like matrix.

One example of a fill component that migrates into a capsule shell is a volatile or semi- volatile chemical, including those that can function as a taggant. Therefore, the present disclosure also contemplates a fill formulation that comprises one or more taggants mixed with one or more polymers. This formulation cars further comprise one or more therapeutic drugs or placebo formulations, depending on the ultimate use contemplated for the administration means comprising the fill formulation.

Taggants are generally recognized as safe (GRAS) compounds including, but not limited to, food substances or components in industrialized countries and agents listed on the FDA inactive ingredient database or a detectable metabolite thereof. A taggant is preferably associated with the active therapeutic drug matrix but is not an integral part of it; that is the taggant is physically located adjacent but preferably not physically integrated into the active therapeutic drug. This physical separation can help address concerns of the taggant negatively altering the stability or therapeutic effect of the administered drag or agent, which is to be avoided. The therapeutic drug itself, or a metabolite of the therapeutic drug can also be a source of taggants. If the therapeutic drug or a metabolite is directly detectable, an additional taggant molecule need not be necessarily added to the fill formulation,

Effective taggants are commonly found in liquid form, although other forms are contemplated within the present disclosure if combination with the selected pharmaceutical polymer results in a mixture that can be loaded into an administration means, such as capsules. Taggants can be utilized in a wide range of concentrations. For example taggants can be used at a dosage of about 1 mg to about i g. Generally, the amount of taggant required to provide detection is directly related to the molecular weight of the molecule being detected. For example, the smaller the molecule being detected in molecular weight, the less taggant is required to provide detection. It should also be noted that the form of the taggant monitored by the methods of the present disclsoure can also be a metabolite of the taggant. While taggants or markers are GRAS compounds or are derived from GRAS compounds, non-GRAS compounds may also be utilized in the fill formulation and dosage forms described herein without departing from the present disclosure.

A taggant is a molecular entity that could either be naturally found in the body (endogenous), or unique (not endogenous to the human body, if the taggant is endogenous it can be detected at concentrations that are readily distinguishable from natural background breath levels during monitoring. If it is unique it can be very easily distinguished in human breath. One embodiments of the present disclosure is directed to the use of GRAS type compounds including those approved as food substances, inactive ingredients and/or excipients. For example, food substances including but not limited to alcohols, esters, aliphatic higher alcohols, aromatic alcohols, thioalcohols, thiols, fatty acids, aliphatic higher aldehydes, aromatic aldehydes, ethers, thioethers, phenol ethers, ketones, phenols, lactones, terpens, aliphatic higher hydrocarbons, furfurais, indoles, and isothiocyanate. Additional examples of suitable compounds include carbonate esters, acetals and ketals. Examples of these chemical classes that are food components in industrialized countries are listed in the Leffmgwell & Associates (Canton, GA) "Flavor-Base 2007." For example, twelve amines are listed by the FDA as chemicals found in food. Archives of regulatory agencies within industrialized countries such as the United States, European Union and Japan can serve as sources for suitable compounds that are food substances, or found in food. In addition, a list of possible taggants is available in U.S. Patent Publication No. 2010/0255598.

A number of compounds, independent of taste or flavor, appear in food because they provide specific roles in food processing and/or are present as by products in the manufacturing process of creating foods. The major functions of food substances include: acidity regulator, anticaking agent, antifoaming agent, antioxidant, bulking agent, carbonating agents, clarifying agents, colloidal agents, color, color retention agent, concentrate, emulsifier, firming agent, flavor enhancer, flour treatment agent, foaming agent, freezant, gelling agent, glazing agent, humectant, liquid freezant, packing gas, preservative, propellant, raising agent, stabilizer, sweetener, and thickener. The use of a chemical to fulfill one of the functions described above, or another function, does preclude its use as a taggant. As mentioned above, the metabolism of many agents such as these to volatile and semi-volatile substances in the breath is known or easily tested in various in vitro and in vivo systems.

Non-limiting examples of preferred GRAS compounds that can be used as taggants include sodium bisulfate, dioctyl sodium sulfosuccinate, polyglycerol polyricinoleic acid, calcium casein peptone-calcium phosphate, botanical compound, ferrous biglycinate chelate, seaweed-derived calcium, docosahexaenoic acid rich single-cell oil, arachidonic acid-rich single-cell oil, fructooligosaccharide, trehalose, gamma cyclodextrin, phytosterol esters, gum arable, potassium bisulfate, stearyi alcohol, erythritol, D-tagatose, and mycoprotein. Possible botanical compounds include chrysanthemum, licorice, jellywort, honeysuckle, lophatherum, mulberry leaf, frangipani, selfheal, and sophoroa flower bud. Many GRAS compounds are in solid form. Thus, preparation of a solution using a suitable solute, preferably one which would not confound the detection mechanisms to be used, is generally preferred. In this way it is possible to dissolve the polymer in the GRAS compound solution in order to reduce volatility, as described herein.

"Olfactory taggants" have distinct odors and thus are particularly suitable for detection in the breath using artificial or electronic noses. The taggant can also preferably include one or more of dimethyl sulfoxide (DMSO), acetaldehyde, acetophenone, trans-Anethole (1-methoxy-4-propyl benzene (anise), benzaldehyde (benzoic aldehyde or bitter almond), benzyl alcohol, benzyl cinnamate, eadinene, camphene, camphor, cinnamaldehyde (3-phenylpropenal), garlic, citronellal, cresol, cyclohexane, eucaiyptol, and eugenoi, eugenyl methyl ether; butyl isobutyrate (n-butyl 2, methyl propanoate (pineapple)); citral (2-trans-3,7-dimethyl-2,6-actadiene- 1 -al); menthol (1-methyl-4-isopropylcyclohexane-3-ol); and alpha-Pinene (2,6,6-trimethylbicyclo-(3,1,1)-2-heptene). Other possible taggants include compounds also commonly referred to as "flavorants", such as n-butanol, particularly 2-butanol (imitation banana) and n-pentanone, particularly 2-pentanone (flavoring derived from apple) and L- or D-carvone, particularly L-carvone (imitation spearmint) and mixtures thereof. While 2-butanol, 2-pentanone have been shown to be effective taggants, other secondary alcohols and ketones are contemplated in accordance with the instant disclosure.

Another class of taggants are molecules that can be detected using means other than detection in the breath and comprise another form of tracer for tracking, in various fields of technology, including biotechnology and the chemical arts, tracers are used to provide information on molecules involved in reaction processes. A tracer is an identifiable substance or property that is introduced into a biological or chemical system and that can be followed through the course of a process to obtain information on the pattern of events in the process or on the reaction or redistribution of molecules or elements involved. Examples of traces are colored dyes and radioactive isotopes. An ideal tracer should be able to provide the information of interest, with minimal or no interference with the process involved. At the very least, such artifacts need to be tracked and factored into the involved process. Frequently, this is an issue in reaction processes, where the tracer is associated with a molecule that has to efficiently react with other reagents in the process. Thus, the present disclosure can be used to deliver the tracer to be used. Examples of well known tracers include isotopes, radioactive labels such as Micro Tracers, dyes, labeled sugar or nucleic acid or protein-based tracers and other means of following chemical reactions.

In the fill formulation of the present disclosure, one or more pharmaceutical polymers are mixed with or dissolved within the one or more fill components. Generally, the polymers are mixed or dissolved within the fill component before loading the component within the administration means, or capsule. Mixing or dissolution of the polymer with the fill component reduces the tendency of the fill component to migrate into or through the capsule shell.

Polymers that can prevent, reduce or delay migration of a fill component into or through a capsule shell are suitable for use in the formulations, capsules, and methods of the present disclosure. The suitable polymer is hydroxypropyl cellulose.

The appropriate polymer for a particular formulation is selected taking into account the desired use of the capsule or fill formulation. For example, if the fill formulation is intended for pharmaceutical use, pharmaceutical grade polymers are preferred, in one aspect, the present disclosure provides approaches for determining the suitability of particular polymers for formulation with particular taggants. More specifically, the methods of preparation, compatibility testing, and stability testing allow for combinations of polymers and taggants disclosed herein to be determined suitable for administration.

The suitable polymer is hydroxypropyl cellulose (HPC) (also known as KLUCEL^{®}, Hercules, Inc.). In general, cellulose ethers are high-molecular weight compounds produced by replacing the hydroxylgroups in the anhydroglucose units of cellulose with alkyl or substituted alkyl groups. Hydroxypropyl cellulose is an ether of cellulose where some of the hydroxyl groups of the cellulose have been hydroxypropylated forming -OCH₂OCHCH₃ groups. As a pharmaceutical polymer, HPC can be used as artificial tears or a lubricant. As a food additive, HPC is generally used as a thickener and an emulsion stabilizer. HPC has now been shown to reduce the volatility of taggants within a fill formulation. The one or more polymers can be utilized in a wide range of concentrations. In certain embodiment the polymer or polymers are present in concentrations ranging from greater than about 0, 1 %. In certain embodiments, the polymer or polymers are present in concentrations ranging from about 0.1 % to about 20.0%. Generally the percent composition is measured by mass if the polymer is a solid and the taggant is a liquid, in certain embodiments the compositions is measured as a volume percent if both the polymer and taggant are liquids. In some embodiments the fill formulation comprises concentrations of 0.5%, 1.25%, and 1.5% polymer (or polymer/taggant). The maximum concentration of polymer is dictated by the maximum viscosity that allows for effective filling of the administration means, or capsule. Exemplary viscosities suitable for effective filling range up to about 2000 to about 3000 mPa.s.

In embodiments where the intended use of the fill formulation is to allow for product tracking, and the product tracking is accomplished using measurement of a taggant in an air sample, the polymer generally reduces the volatility of the taggant, which in turn reduces migration of the fill component into the capsule, in one embodiment, the air sample is taken from a patient's breadth. Reduction in the volatility of the taggant or tracker allows a sufficient concentration of the taggant to have an effective shelf-life so it can be successfully detected in the patient sample after administration in combination with the therapeutic drag or agent. In certain embodiments, determination of a patient's compliance with a drug dosing regimen depends from the ability to detect the presence of the taggant in the sample taken from the patient.

The fill formulation comprises hydroxpropyl cellulose, and at least compound selected from the group consisting of 2-butanol, 2-pentanone, and L-carvone, and mixtures thereof. It is disclosed a fill formulation according to the present disclosure comprises hydroxpropyl cellulose, and at least one volatile compound selected from the group consisting of 2-butanol, 2-pentanone, and L-carvone, and mixtures thereof. The at least one volatile compound is present in amount ranging from from about 80% to about 99.9 % (w/w). In one embodiment, the polymer is hydroxypropyl cellulose provided in an amount ranging from about 0.1 % to about 20 % (w/w).

The present disclosure is further directed to the loading of the fill formulation into an administration means. In certain embodiments the administration means is one or more capsules. The fill formulations of the present disclosure are suitable for both hard capsules and soft capsules.

Generally, "capsule" refers to both empty and filled capsules whereas "shell" specifically refers to an empty capsule. In case the hard capsule shells are filled with substances in liquid form, it is intended that the hard capsules of the disclosure may be optionally sealed or banded according to conventional techniques to avoid leakage of contained substances and to reduce air exposure of the contents.

As used herein "hard capsule" refers to a conventional hard pharmaceutical capsule intended for oral administration to a human or animal being. The hard capsules of the present disclosure do not structurally depart from the conventional definition of hard capsules. Hard capsules generally consist of two co-axial, telescopically-joined parts, referred to as body and cap. Normally, caps and bodies have a side wall, an open end and a closed end. The length of the side wall of each of said parts is generally greater than the capsule diameter. The capsule caps and bodies are telescopically joined together so as to make their side walls partially overlap and obtain a hard capsule shell. In certain embodiments, the side walls of caps and bodies have substantially the same length so that, when a cap and a body are telescopically joined, the side wall of said cap encases the entire side wall of said body.

Hard pharmaceutical capsules are generally manufactured by using a dip molding process. In tills process, pin molds are dipped into an aqueous-based film forming composition. A film is formed by subsequently gelling the composition adhered on the pins. The film is then dried, stripped off the pins and cut to a desired length. Thus, capsules caps and bodies are obtained that can later be filled with a substance and telescopically joined together such that a filled, hard pharmaceutical capsule is obtained. This process is generally described in U.S. Patent Nos. 5,264,223, 5,756,123 and 5,756, 123.

In certain embodiments one or more water-soluble film forming polymers are the primary constituent by weight of the capsule shells according to the present disclosure, in certain embodiments, the water soluble ftim forming polymers are selected from cellulose derivatives, such as HPMC, gelatin, pullulan, polyvinyl alcohol and starch derivatives, such as hydroxypropyl starch. In one embodiment, the water soluble film forming polymers are selected from HPMC, gelatin, pullulan, polyvinyl alcohol and hydroxypropyl starch. These polymers form films with suitable mechanical performance in terms of elastic module and brittleness.

in one embodiment, the film forming polymers comprise HPMC, gelatin, and combinations thereof. Suitable types of HPMC are HPMC type 2930 (as defined in USP30-NF25), HPMC 2208 and HPMC 2906 (as defined in USP30-NF25) and mixtures thereof. In one embodiment the film forming polymer is HPMC 2910. In another embodiment, the film forming polymers consist of gelatin. Gelatin is generally available in two types - type A produced through acid processing and type B produced through alkali processing. Either one of these types or mixtures thereof are suitable for the present capsule shell compositions. Other film forming polymers that can be used in the capsules of the present disclosure include starch, starch derivatives other than hydroxylpropyl starch, pectin, cellulose, celluloses derivatives other than HPMC and mixtures thereof. Also contemplated within the film-forming polymers for use in the present capsules are bacterial or yeast-derived film-forming polymers (exo-polysaccharides) such as the pullulan mentioned above. Other typical exo-polysaccharides are xanthan, acetan, gellan, welan, rhamsan, furcelleran, succinoglycan, scleroglycan, schizophyllan, tamarind gum, curdlan, dextran and mixtures thereof.

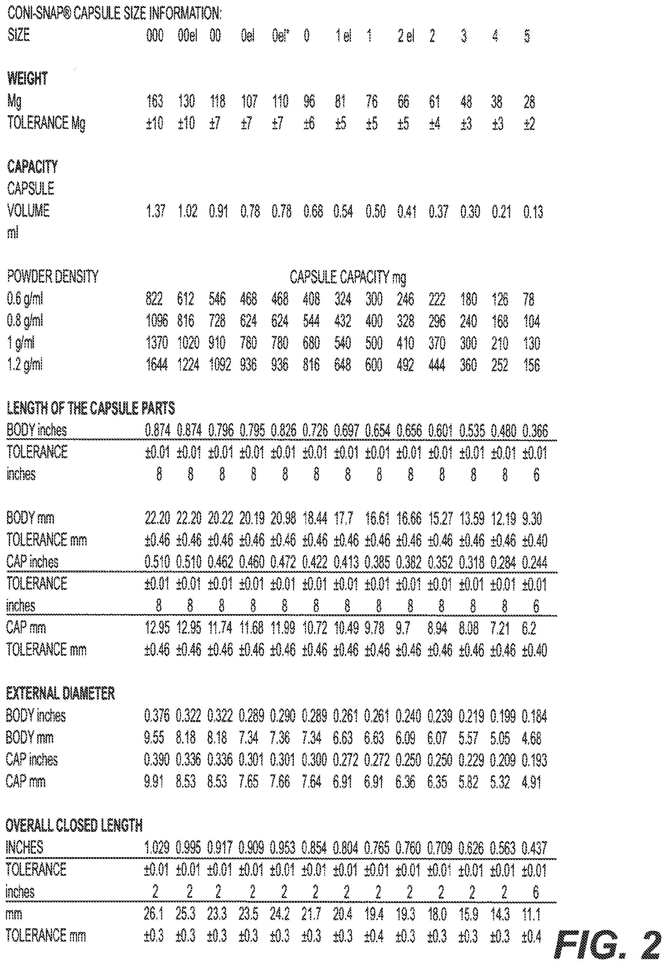

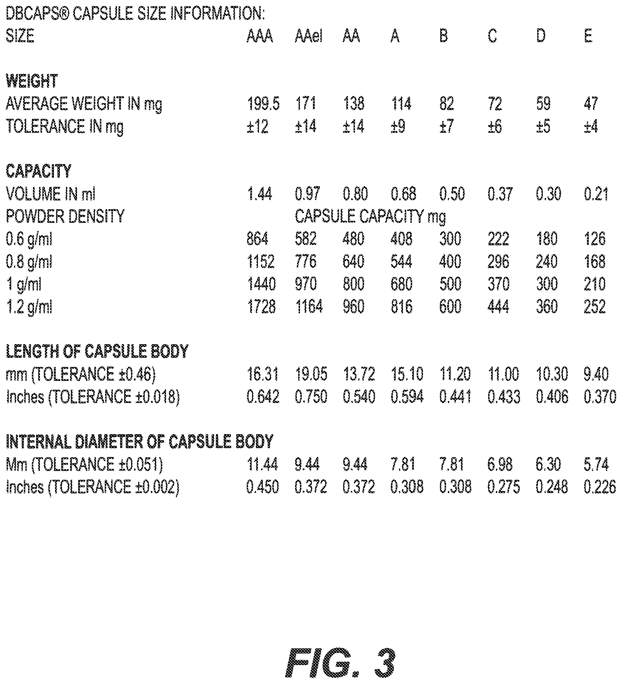

An additional optional constituent of capsules shells can be one or snore anti-cross linking agents such as ethylenediaminetetraacelic acid (EDTA). If the agent responsible for the cross linking is the same as the taggant according to the present disclosure the need for anti-cross linking components in the shell may be reduced. Other optional components of the capsule compositions include surfactants (such as sodium lauryl sulfate), dyes, and preservatives (such as methylparaben and propylparaben). Exemplary gelatin capsules for use in accordance with the disclosed fill formulation include LICAPS^{®} liquid filled capsules, DBCAPS^{®} two-piece gelatin or hydroxypropylmethyl cellulose capsules, and CONI-SNAP^{®} hard gelatin capsules from Capsugel, Inc. Exemplary non-gelatin capsules for use with the instant fill formulations include Vcaps^{®} Plus capsules from Capsugel, Inc. Figures 1 -3 provide sizing and characteristics of exemplary Capsugel capsules suitable for use with the fill formulation and in accordance with the instant disclosure.

As used herein "soft capsule" refers to a conventional soft pharmaceutical capsule intended for oral administration to a human or animal being, said capsule commonly consisting of a single unit. The soft capsules of the present disclosure do not structurally depart from the conventional definition of soft capsules. These single units can be manufactured using various processes. One standard method is the use of two continuous ribbons of shell constituent, such as gelatin, which are formed and brought together between a pair of revolving dyes and an injection wedge. Insertion of the fill composition under pressure and sealing of the soft capsule occur simultaneously and the two processes are precisely coordinated. The process of sealing also separates the formed, filled soft capsules. The rotary die machine can also consist of multiple injection pumps allowing multiple capsules to be prepared at once. Accuracy of injection of the proper amount of fill solution and capsule weight can he periodically determined by analytical balances associated with the machine. Patent literature disclosing this process includes, for example, U.S. Patent Application Publication No. 2003/0138483. Exemplary soft capsules include SGCAPS^{®} soft gelatin capsules from Capsugel, Inc.

In certain embodiments one or more water-soluble film forming polymers are the primary constituent by weight of the soft capsule shells of the present disclosure. As described above, the use of water-soluble polymers in an injection-based manufacturing process for soft pharmaceutical capsules is known in the art. In one embodiment, the one or more water-soluble film forming polymers are gelatin obtained from skin and bone of bovine and porcine sources. Acid or basic preparations of gelatin can be used as ingredient in the soft shell composition. Either one of these types or mixtures thereof are suitable for the present capsule shell compositions. Another component of traditional soft pharmaceutical capsules is a plasticizing agent. Some commonly used plasticizing agents include, but are not limited to sortbitol, sorbitan, glycerol, xylitol, polyglycerol, propylene glycol, glucose, fructose, polyols, for example macrogol 400, 600, 1200, 1500, 200, or 400, macrogols between 400 and 4000,
or polyoxamers. One possible mixture of plasticizing agents is sorbitans, mannitol, and hydrogenated saccharides. This can be used alone, or in combination with other plasticizing agents such as glycerol. The soft capsule composition can also comprise other components such as anti-cross-Iinking agents, opacifiers, dyes or excipients, flavors, sweeteners, and preservatives.

Although the various film forming polymers have been described above generally for hard or soft capsules, shell constituents disclosed in this application may be used for both hard and soft capsule compositions.

As noted, the present disclosure contemplates filling the fill formulation into hard or soft capsules. Other formulation architectural approaches are also contemplated including, but not limited to, multi-phase capsules, multi-component capsules, double capsules, capsule-in-capsule ("cap-in-cap") systems. A capsule in accordance with the present disclosure may comprise an inner and outer capsule wherein the inner capsule comprises the fill formulation and the outer capsule comprises the inner capsule and a further component such as, for example, a therapeutic drug and a placebo formulation. Capsules in accordance with the present disclosure may comprise an inner and an outer capsule wherein the inner capsule comprises a component such as, for example, a therapeutic drug and a placebo formulation and the outer capsule comprises the inner capsule and fill formulation. In alternative embodiments, the inner and outer capsules have the same composition. In other embodiments, the inner and outer capsules have different compositions. In certain embodiments, an inner capsule may be freely contained within an outer capsule, the outer capsule which may or may not include a liquid (e.g., lipid) fill. In certain embodiments, an inner capsule can be adhered or fixed to the interior of a larger capsule. Furthermore, a separation or barrier, such as for example but not limited to a film layer may be incorporated within the capsule. Thus, in one aspect, the present disclosure provides a method of avoiding migration of fill into a capsule shell, comprising the steps of mixing the fill component with polymer to form a gel-like matrix and loading the mixture into the capsule shell.

Capsules in accordance with the instant disclosure may be prepared for all enteral routes of administration, including but not limited to oral and rectal administration. Furthermore, the fill formulation according to the instant disclosure, and capsules containing said fill formulation, may be useful for pharmaceuticals, veterinary products, nutraceuticals, vitamins, and dietary supplements, foodstuffs, cosmetic products and flavoring agents. The fill formulation and capsules containing said fill formulation in accordance with the instant disclosure may also optionally include an active ingredient, such as a therapeutic drug or an API, within the capsule. The API may be any such API known to those of ordinary skill in the art and selected according to the desired formulation of the skilled artisan.

Various examples are provided which demonstrate the stability of fill formulations comprising volatile compounds entrapped in a gel-like structure filled in LICAPS^{®} and other capsules to prevent loss of the volatile compounds from the capsule.

### Examples

The following examples are merely illustrative, and should not be construed as limiting the present disclosure

### Example 1: Baseline: Volatile Taggant Without Entrapment to a Gel Like Structure: 2-

### Example 1 : Baseline: Volatile Taggant Without Entrapment in a Gel Like Structure: butanol alone

Preparation LICAPS^{®} capsules were manually filled with the composition described in Table 2.

Compatibility testing: Compatibility testing was executed and softening of the capsules was observed after 3 months storage at 40"C and 75% relative humidity (RH).

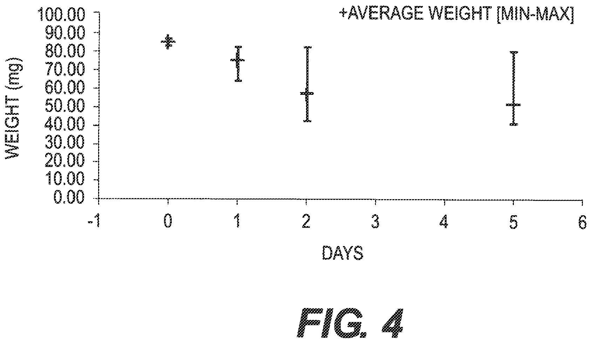

Stability testing (loss of volatile compound as measured by weight decrease): Six (6) LICAPS^{®} Capsules were manually filled with 2-butanol. Each LICAPS^{®} capsule was individually weighed at time zero, stored in a ventilated oven at 40°C, and then reweighed at defined time points. After 5 days, a significant decrease in weight was observed indicating loss of the 2-butanol. The results are represented graphically in Figure 4.

A mechanical robustness test was performed to evaluate the tendency for capsule brittleness when filled with 2-butanoi as a function of the relative humidity of the ambient air. Results of the mechanical robustness test are presented in Table 3.

| Constituent | Unit formulation |
|---|---|
| **2-butanol** | **40 mg** |

The mechanical test results for LICAPS^{®} capsules filled with 50µL of 2-butanol were within acceptable parameters. However, for a percentage of loss on drying (LOD) at 12.6 and 15.4%, the percentages of broken capsules (20%) were unacceptable.

The mechanical test results for the LICAPS^{®}capsules filled with 225 µL of 2-butanol were not within acceptable parameters at low relative humidity (8% LOD) but were within acceptable parameters for a percentage of LOD higher than 10%.

These results indicate a change in the mechanical properties of the shell leading to an embrittlement of the capsule shell for the size 3 LICAPS^{®} capsules filled with 2-butanol.

### Dissolution test results after storage at 40°C/75% RH

The dissolution test is a further step to determine the behavior of filled unsealed LICAPS^{®} capsules stored under accelerated conditions.

Dissolution test results for LICAPS^{®} capsules stored for 3 weeks, 3 and 6 months with a fill composed of 50µL or 22.5 µL of 2-butanol, emptied by removal of the fill at the above time points, and filled with acetaminophen as a reference substance, are presented in Table 4a and 4b

**Table 4a: Dissolution test results in demineralized water for size 3 UNCAPS^{®} capsules filled with 50µL of 2-butanol (n=6 except for the 6 months time point n=5)**

| | **% Acetaminophen dissolved at 45 min after storage at 40 °C/75% RH** | | |
|---|---|---|---|
| **Specifications** | | **> 80%** | |
| **Percentage** | **Minimal** | **Average** | **Maximal** |
| After 3 weeks | 95.0 | **98.2** | 99.7 |
| After 3 months | 91.7 | **94.3** | 97.0 |
| After 6 months | 90.8 | **96.1** | 98.8 |

**Table 4b: Dissolution test results in demineralized water for size 3 LICPAS^{®} capsules filled with 225 µL of 2-butanol (n=6)**

| | **% Acetaminophen dissolved at 45 min after storage at 40 °C/75% RH** | | |
|---|---|---|---|
| **Specifications** | | **> 80%** | |
| **Percentage** | **Minimal** | **Average** | **Maximal** |
| After 3 weeks | 90.4 | **97.2** | 100.0 |
| Alter 3 months | 98.5 | **99.7** | 99.7 |
| After 6 months | 90.1 | **96.2** | 100.0 |

For the size 3 LICAPS^{®} capsules filled with 50µL of 2-butanol, the dissolution test results in demineralized water after 3 weeks, 3 and 6 months storage at 40°G75% RH were within acceptable parameters as the average percentage of,
acetaminophen dissolved at 45 minutes is greater than 80%. No interaction was noticeable between 2-butanol and the gelatin shell at the 3 weeks time point.

During preparation of the capsules for the dissolution testing (emptying the fdl from the capsules and filling the empty capsules with Acetaminophen), a softening of the capsules was observed at the 3 months time point. The same observation (softening) was confirmed at the 6 months time point.

Even if the dissolution test was within acceptable parameters, the softening of the capsules observed demonstrates that 2-butanol has the tendency to weaken the capsule shell.

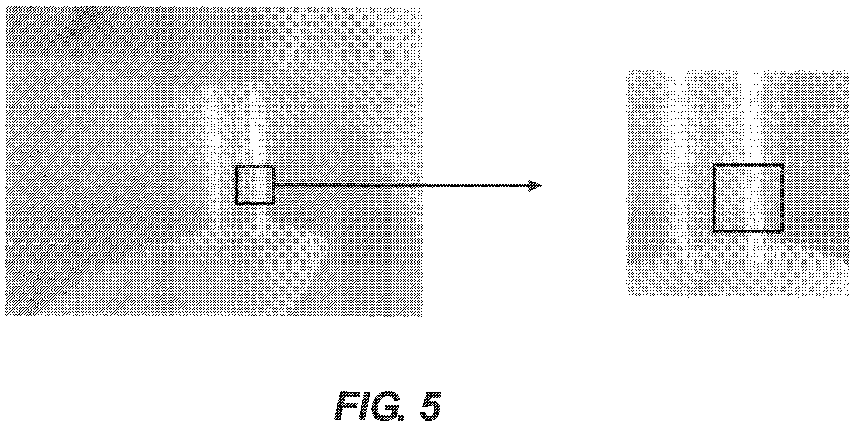

For the size 3 LICAPS^{®} capsules filled with 225 µL of 2-butanol, the dissolution test results in demineralized water after 3 weeks, 3 and 6 months storage at 40°C/75%RH were within acceptable parameters as the average percentage of acetaminophen dissolved at 45 minutes is greater than 80%. However a deformation of the LICAPS^{®} capsules was observed at the 3 months time point. Figure 5 shows the deformation observed after 3 months storage at 40°C/75%RH. The deformation confirms that 2-butanol has the tendency to weaken the capsule shell

The results of the hygroscopicity test for LICAPS^{®} capsules filled with 2-butanol were not within acceptable parameters at storage conditions below 30%RH and above 60%RH for a filling of 50µL of 2-butanol and below 20% RH and above 55% RH for a filling of 225µL of 2-butanol.

2-butanol appeared to present a hygroscopic propensity at high relative humidity conditions which could influence the water content of the capsule shell. 2-butanol is considered as a hygroscopic material, (www.chemicalbook.com)

For the mechanical robustness test, the results were within acceptable parameters for the capsules containing 50µL of 2-butanol. For the capsules containing 225 µL of 2-butanoi, the results were not within Capsugel's recommendations for an 8% LOD but within acceptable parameters for a percentage of LOD at 10%.

After 3 weeks, 3 months and 6 months at 40°C/75% RH, dissolution testing resulted in demineralized water were within acceptable parameters for LICAPS^{®} capsules filled with both quantities of 2-butanoi. However, softening of the capsules was observed for capsules filled with 50µL of 2-butarioi and there appeared a slight deformation for the LICAPS^{®} capsules filled with 225 µL of 2-butanol from the 3 months time point onwards.

It was concluded that 2-butanol weakened the capsule shell of LICAPS^{®} size 3 capsules due to embrittlement of the capsules for both quantities of 2-butanol tested and softening or deformation of the capsules was observed under accelerated conditions from the 3 months time point onwards.

Materials: In evaluating the stability of of 2-butanol filled into LICAPS^{®} capsules the study was initiated in standard transparent size 3 LICAPS^{®} capsules (batch 70429891) available from Capsugel. 2-butanol was obtained from SIGMA Aldrich (batch: SZBA0540V).

Methods: Size 3 LICAPS^{®} capsules (n=6) were manually filled with 2-butanol (50µL or 225 µL). The LICAPS^{®} capsules were either not sealed or sealed on CPS^{®} equipment. Each capsule was individually weighed at time zero and placed in an oven at 40°C and reweighted at defined time points.

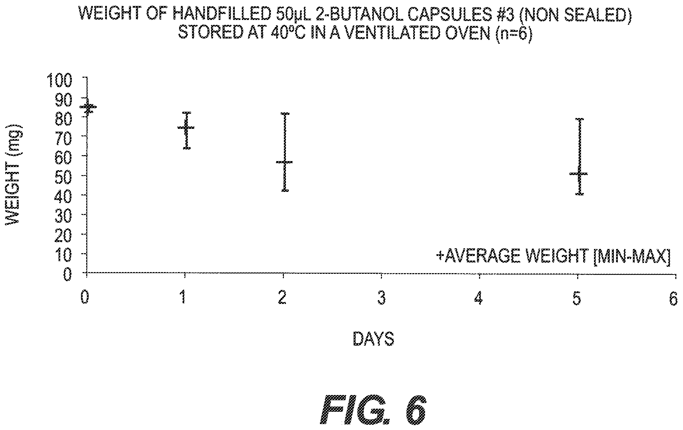

Figure 6 shows the evolution of the weight of non-sealed size 3 LICAPS^{®} capsules manually filled with 50µL of 2-butanol in function of the days of storage in an oven at 40°C. The weight of empty size 3 LICAPS^{®} capsules is 48 ± 3 mg.

An important decrease in weight of the non sealed capsules containing 50µL and consequently of the quantity of 2-butanoi could be observed after 5 days storage at 40°C in the oven.

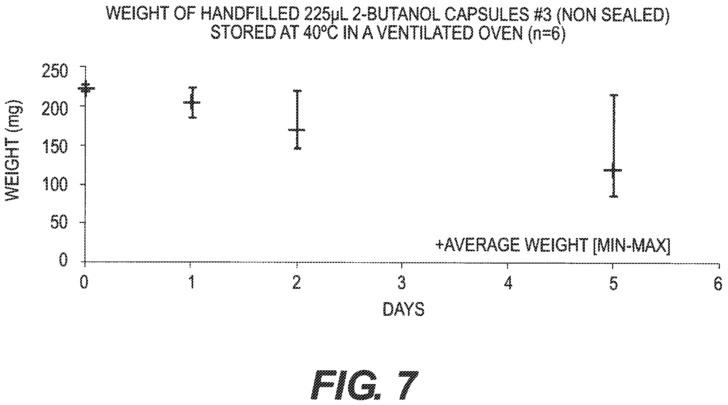

Figure 7 shows the evolution of the weight of non-sealed size 3 LICAPS^{®} capsules filled with 225µL of 2-butanol in function of the days of storage in an oven at 40°C.

Similar to Figure 6, an important decrease in weight and consequently of the quantity of 2-butanol was observed after 5 days storage at 40°C in the oven.

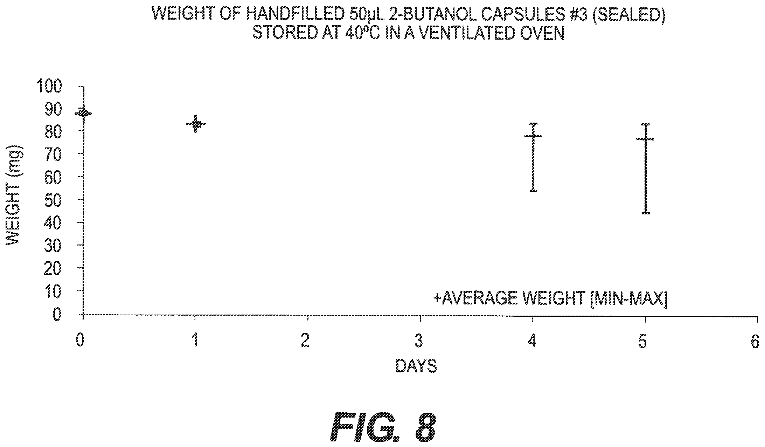

Figure 8 shows the evolution of the weight of the size 3 LICAPS^{®} capsules filled with 50µL of 2-butanol and sealed using CFS^{®} equipment in function of the days of storage in an oven at 40°C.

A decrease in weight and consequently of 2-butanol was observed in function of time and in particular for one capsule but the decrease was less pronounced than the one observed for the non sealed capsules. The sealing was performed on CPS^{®} equipment but no sealing integrity testing was performed (depression chamber testing) and therefore, one capsule might have a poor sealing or the sealing is not adapted to retain volatile components such as 2-butanol.

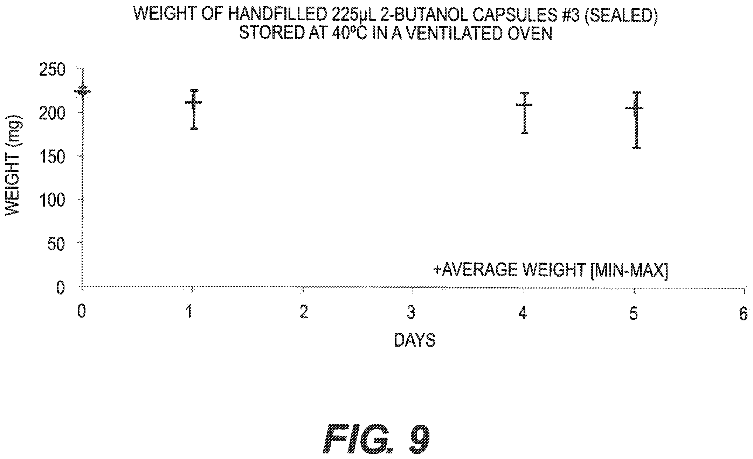

Figure 9 shows the evolution of the weight of the size 3 LICAPS^{®} capsules filled with 225 µL of 2-butaiiol and sealed using CPS^{®} equipment in function of the days of storage in a 40°C oven.

A small decrease in weight and consequently of the quantity of 2-butanol was observed in function of time and in particular for one capsule but the decrease was less pronounced than the one observed for the non sealed capsules. The sealing was performed on CPS^{®} equipment but no sealing integrity testing was performed (depression chamber testing) and therefore, one capsule might have a poor sealing or the sealing is not adapted to retain volatile components such as 2-butanol,

Based on the results obtained for 2-butanol with the LICAPS^{®} capsules, it was concluded that 2-butanol weakens the capsule shell of the capsules due to embrittlement of the capsules for both quantities of 2-butanol tested and a softening or deformation of the capsules was observed under accelerated conditions from the 3 months time point onwards. Example 2 - Addition of Pharmaceutical Polymers to 2-butanol

A variety of polymers were tested to observe solubilization in 2-butanol and the formation of an acceptable gel structure. The results of these tests are illustrated in Table 5.

Compositions comprising povidone, HPMC, xanthan gum and silica are reference examples.

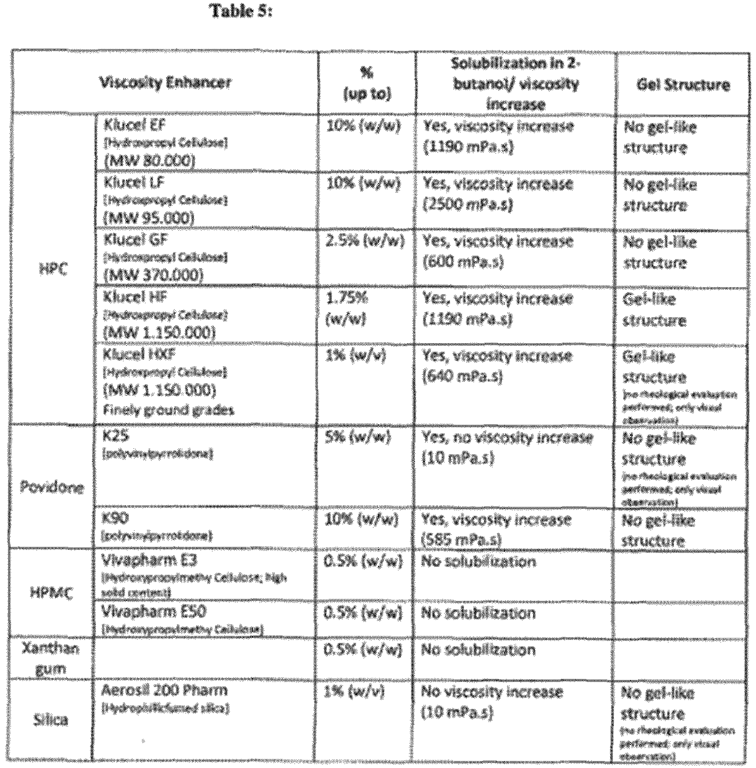

The effect of the addition of HPC on the viscosity of 2-hutanol was evaluated. The 2-butariol was obtained from SIGMA Aidrich (batch: SZBA0540V) and the HPC (grade Klucel HXF Pharm. batch 03231) from Hercules.

Methods; Different quantities of HPC were slowly added to 2-butartol under magnetic mixing at 500 rpm. After 4 hours magnetic stirring, the solution was left overnight without mixing and the solution was checked visually for the absence of any HPC particles. The viscosity of the solution was then measured using a Mars II Haake system from Thermo Fischer.

Results of Addition of HPC : Three different quantities of HPC (w/v) were added to pure 2-butanol (0.5%, 0.75% and 1 %).

After visual observation the next day, no HPC particles were visually observed. A gel like structure was obtained after addition of 0.75 % HPC and 1 % HPC.

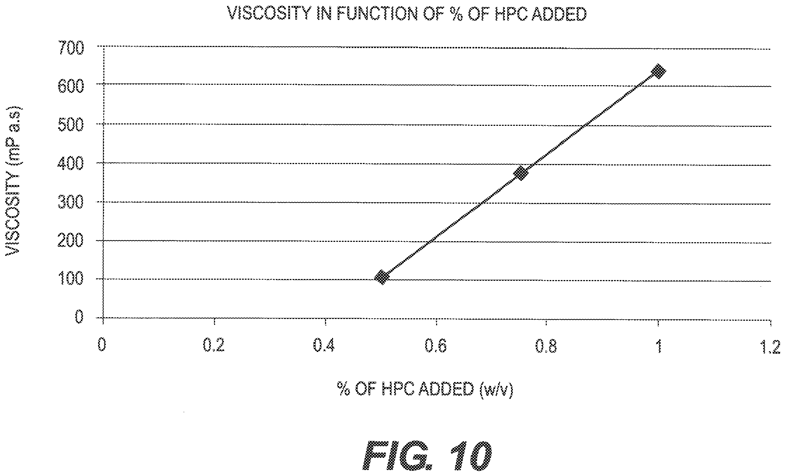

The viscosity of the three mixtures was then measured (n=1). Table 6 and Figure 10 summarizes the viscosity as a function of the quantity of HPC added. 1

| Constituent | Unit formulation |
|---|---|
| **2-butanol** | **40 mg** |
| **HPC** | **0.3 mg** |

A linear correlation was observed between the percentage of HPC added to the solution of 2-butanol and the viscosity measured at 21 °C. For all the quantities tested, the viscosities were within acceptable parameters for filling on small laboratory scale and/or commercial scale capsule filling equipment, i.e, 300-1000 mPa.s. The addition of HPC allowed obtaining a gel like structure

0.75 % of HPC (w/v) added to the solution of 2-butanol was selected for further development due to the gel like structure observed and the viscosity value obtained. Example 3; Addition of Pharmaceutical Polymer to Volatile Taggant in LICAPS^{®}

2-butanol was entrapped in a 0.75% Hydroxy Propyl Cellulose (HPC) gel like structure.

| **Formulation** | **Viscosity (mPa.s) at 21°C** |
|---|---|
| 2-butanol + 0.5% HPC | 109 |
| 2-butanol + 0.75% HPC | 380 |
| 2-butanol + 1% HPC | 641 |

Preparation: HPC was slowly added to the 2-butanol under magnetic stirring during 4 hours. The mixture was then left overnight without magnetic stirring until complete solubilization of the HPC (visual observational check).

The LICAPS^{®} capsules were manually filled with the composition described in Table 7.

Compatibility testing: Compatibility testing was executed and no softening of the capsules was observed after 3 months storage at 40°C/75%RH.

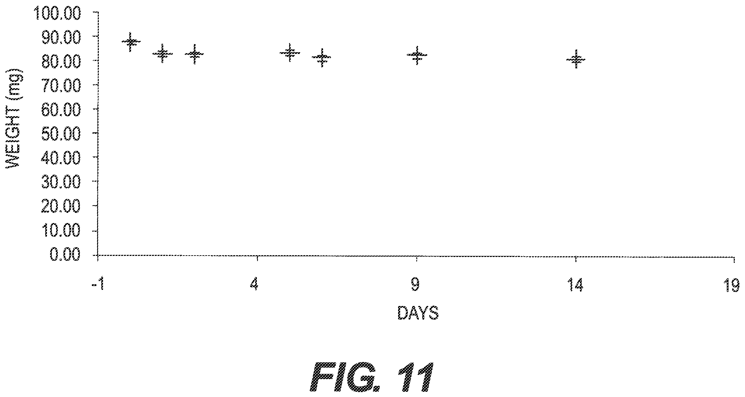

Stability testing (loss of volatile compound as measured by weight decrease): Six LICAPS^{®} capsules were manually filled with the composition described in Table 7. Each LICAPS^{®} capsule was individually weighed at time zero, stored at 40°C in a ventilated oven, and reweighed at defined time points. The results are represented graphically in Figure 1 1. As can be seen through comparison of Figure 4 to Figure 11 , the decrease in weight after 34 days for the capsules containing 2-butanol entrapped in the gel like structure was less than the decrease in weight observed after 5 days for capsules containing 2-butanol which was not entrapped in the gel like structure.

### Compatability Study of 2-Butanol + 0.75% (w/v) HPC with LICAPS^{®} Capsules

Materials; Capsugel evaluated the compatibility of 2-butanol + 0.75% (w/v) HPC with LICAPS^{®} capsules. The compatibility study was initiated in transparent size 3 LICAPS^{®} (batch 70429891) capsules supplied by Capsugel. 2-butanol was obtained from SIGMA Aldrich (batch: SZBA0540V) and HPC (Klucel HXF Pharm. batch 03231 ) was obtained from Hercules.

Methods: Compatibility testing was performed according to the methods described hereinabove.

### Results of compatibility study with white opaque size 3 LICAPS^{®} capsules

The compatibility testing was initiated by filling around 50µL of 2-butanol +0.75% (w/v) HPC in size 3 LICAPS^{®} capsules. The compatibility study results are summarized in Figure 12 and Tables 8, 9 and 10.

### Hygroscopicity test

Results of the hygroscopicity test are presented Figure 12 and in Table 8 which shows hygscopicity test results after 2 weeks storage of LICAPS^{®} filled with 2-butanol + 0.75% (w/v) HPC at room temperature (RT)

**Table 8:**

| **Relative humidity storage conditions** | **Variation of weight (water exchange) in %** | |
|---|---|---|
| | **25% RH** | **65% RH** |
| **Recommendation** | **-2%** | **+2%** |
| 50 µL of 2-butanol + 0.75% HPC | -9.5 | 3.8 |

The results of the hygroscopicity test for LICAPS filled with 2-butanoi + 0.75%(w/v) HPC were not within acceptable parameters for transparent size 3 LICAPS^{®} capsules tested at storage conditions below 25% RH and above 60% RH. Moreover, the data in Figure 12 shows that the water uptake is not stabilized after 2 weeks of storage. However, no deformation of the capsules was observed.

### Mechanical robustness, test

Results of the mechanical robustness test for the formulation of 2-butanol 0.75% HPC with LICAPS^{®} size 3 capsules are presented in Table 9.

**Table 9: Mechanical resistance test results**

| **Recommendation for natural transparent capsules** | **% Broken capsules after 3 weeks storage for** | |
|---|---|---|
| | **8% LOD** | **10%LOD** |
| | **< 50%** | **< 30%** |
| 50 µL 2-butanol + 0.75% HPC | 33 | 12 |

The mechanical test results for she LICAPS^{®} capsules filled with 50µL of 2-butanol + 0.75% HPC were within acceptable limits.

The mechanical robustness was improved compared to pure 2-butanol without HPC filled in LICAPS^{®} capsules.

### Dissolution test results after storage at 40°C/75% RH

Dissolution test results for LICAPS^{®} capsules stored for 3 weeks, 3 and 6 months with a fill composed of 50µL of 2-butanol + 0.75% HPC, emptied by removal of the fill at the above time points, and refilled with acetaminophen as a reference substance, are presented in Table 10 which sets for dissolution test results in demineralized water for size 3

LICAPS^{®} capsules filled with 50µL of 2-butanol + 0.75% HPC (n=6).

**Table 10**

| | **% Acetaminophen dissolved at 45 min after storage at 40 °C/75% RH** | | |
|---|---|---|---|
| **Specification** | | **> 80%** | |
| **Percentage** | **Minimal** | **Average** | **Maximal** |
| After 3 weeks | 95.3 | 98.7 | 99.7 |
| After 3 months | 91.6 | 96.5 | 99.4 |
| After 6 months | 86.0 | 91.4 | 97.6 |

For the size 3 LICAPS^{®} capsules filled with 50uL of 2-butanol + 0.75% HPC, the dissolution test results in demineralized water after 3 weeks, 3 and 6 months storage at 40°C/75% RH were within acceptable parameters as the average percentage of acetaminophen dissolved at 45 minutes is greater than 80%. No softening of the capsule shell was observed.

Adding HPC in the formulation improved capsule integrity by preventing the migration of 2-butanol into or through the capsule shell.

### Stability of LICAPS^{®} Capsules Filled with 2-Butanol + 0.75% HPC

Materials: The stability of 2-butanol + 0.75% HPC filled into LICAPS capsules was evaluated. The study was initiated in transparent size 3 LICAPS^{®} capsules (hatch 70429891) available from Capsugel. The 2-butarjol was from SIGMA Aidrich (batch: SZBA0540V) and the HPC (batch 03231) from Hercules.

Methods: LICAPS^{® size 3 capsules (n=6) were filled with a formulation of 2-butanol +0.75% HPC (50 µL).} The LICAPS^{®} capsules were either not sealed or manually sealed. Each capsule was individually weighted at time zero and placed in a oven at 40°C for a week and then reweighted at defined time points.

Results: Figures 13 and 34 show the evolution of the weight of the size 3 LICAPS^{®} capsules non sealed and sealed filled with 50µL of 2-butanol sealed and non sealed as a function of the days of storage at 40°C.

For both sealed and non sealed capsules a slight decrease of the weight of the capsules was observed overtime after storage in a ventilated oven at 40°C. The decrease observed was much more limited than for the formulation which did not contain HPC even for the non sealed capsules. The addition of HPC in the formulation improved the stability.

Based on the results obtained for 2-butanoi + 0.75% HPC filled in size 3 LICAPS^{®} capsules it was concluded that adding the HPC to the solution of 2-butanol improved the weight stability compared to a fill containing only 2-butanol without HPC.

The addition of povidone and silica was insufficient to increase the viscosity of the 2-butanoi solution at the quantities tested to obtain a gel like structure.

### Example 4: Mixture of Taggants Within Pharmaceutical Polymer

The effect of the addition of HPC on the viscosity of the formulation containing 2-butanol, 2-pentanone and L-carvone was evaluated. The 2-butanol was from SIGMA Aidrich (batch: SZBA0540V), the 2-pentanone from SIGMA Aldrich (batch MKBG 1293V), L-carvone from Sigma Aidrich (batch: MKBF6983V) and the HPC (Klucel HXF Pharm. batch 03231) from Hercules.

In this evaluation, 2-butanol + 2-pentanone + L-carvone (Formulation 4) was entrapped in a 1.5% HPC gel like structure and filled in a sealed LICAPS^{®} capsule, as shown in Table 1 1 .

**Table11:**

| Constituent | Unit formulation |
|---|---|
| **2-butanol** | **60 mg** |
| **2-pentanone** | **60 mg** |
| **L-carvone** | **30 mg** |
| **HPC** | **2.25 mg** |

Preparation: 2-butanol was mixed with 2-pentanone using a magnetic stirring in a glass bottle. L-carvone was then added to the mixture. When the mixture was visually homogeneous, HPC was slowly added under magnetic stirring. After around 4 hours of mixing the formulation was left overnight until complete solubilization of the HPC. Complete solubilization was visually checked.

Compatibility testing: Compatibility testing was executed according to the standard compatibility testing program described in the previous Examples. No softening of the capsules was observed after 3 months storage at 40 °C/75%RH.

Stability study (less of volatile compound as measured by change in assay of the volatile compound): 2-butanol was mixed with 2-pentanone using a magnetic stirring in a glass bottle. L-carvone was then added to the mixture. When the mixture was visually homogeneous, HPC was slowly added under magnetic stirring. After around 4 hours of mixing the formulation was left overnight until complete solubilization of the HPC. Complete solubilization was visually checked.

### Hygroscopicity test

Results of the hygroscopicity test are presented in Table 12 and in Figure 15.

**Table 12: Hygroscopicity test results after 2 weeks storage of LICAPS^{®} filled with formulation 4 +1.5% HPC at room temperature (RT)**

| **Recommendation** | **-2%** | **+2%** |
|---|---|---|
| Formulation 4+1.5% HPC | -4.4 | 3.4 |

Figure 17 illustrates that t the water uptake seems stabilized after 2 weeks of storage and no deformation of the capsules was observed.

### Mechanical robustness test

Results of the mechanical robustness test for the formulation 4 + 1.5% HPC with LICAPS^{®} size 3 capsules are presented in Table 13.

**Table 13: Mechanical resistance test results**

| **Recommendantion for natural transparent capsules** | **% Broken capsules after 3 weeks storage for** | |
|---|---|---|
| | **8% LOD** | **10% LOD** |
| | **< 50%** | **< 30%** |
| Formulation 4 + 1.5% % HPC | 18 | 6 |

The mechanical test results for the LICAPS capsules filled with formulation 4 + 1.5% HPC were within acceptable limits.

In a technical batch stability study. Formulation 4 as set forth in Table 1 1 above was filled into LICAPS^{®} capsules and sealed using a lab- scale filling and sealing equipment (e.g., CPS^{®} Liquid Filling & Sealing Machine).

One part of the capsules were, put in polyethylene (PE) bags and placed on stability at 25°C/60%RH and another part of the of capsules were packaged in PET/Alu/PE (polyethylene terephthalate/aluminum/polyethylene) packs and placed on stability at 25°C/60%RH and 40°C/75%RH.

2-butanol and 2-pentanone assay were determined at defined time points by gas chromatography. After 6 months storage at 25°C/60% RH in PE bags, the results of the assay for 2-butanol and 2-pentanone had not changed substantially compared to the time, zero time point. (% label claim of respectively 101.3 and 100,7%)

After 6 months storage at 25°C/60% RH in PET/Alu/PE bags, the results of the assay for 2-butanol and 2-pentanone had not changed substantially compared to the time zero {% label claim of respectively 100.5 and 100.3%)

After 6 months storage at 40°C/75% RH in PET/Alu/PE bags, the results of the. assay of 2-butanol and 2-pentanone had not changed substantially compared to the time zero time point. (% label claim of respectively 99.7 and 99.8%)

After 6 months, the assays indicated that during storage no substantial loss of 2-butanol or 2-pentanone occurred and this independently of the packaging or storage conditions. Therefore, it can be concluded that the fill formulation including entrapment of volatile taggants within a get like structure in a sealed LICAPS^{®} capsule prevents loss of volatile taggant from the capsule.

### Example 5: Addition of Pharmaceutical Polymers to Peppermint Oil and Thyme Oil

Preparation method: Peppermint oil or thyme oil and the pharmaceutical polymer (e.g. HPC, HPMC, Povidone, Copovidone, silica, xanthan gum) are mixed at room temperature and homogenized using magnetic stirring for 4 hours in order to achieve complete solubilization of the polymer. The mixture is then maintained at room temperature overnight without any stirring in order to obtain a gel-like structure.

Conclusion: The addition of HPC allowed to obtain gel-like structures for thyme oil based formulations (i.e. Klucel^{®} GF and Klucel^{®} HXF for concentrations up to 4% w/w) and for peppermint oil based formulations (i.e. Klucel^{®} LF for concentrations up to 10%, Klucel^{®} GF up to 8% and Klucel^{®} HXF up to 5% w/w) and these gel-like structures could serve, as fill formulations preventing migration of peppermint or thyme oil into or through the capsule shell.

Table 14 illustrates the results of testing a variety of polymers to illustrate optimal solubilization in peppermint oil and the formation of an acceptable gel structure.

Compositions comprising povidone/ copovidone, HPMC, silica, xanthan gum are reference examples.

| **Viscosity Enhancer** | | **% (w/w)** | **Solubilization in peppermint off/viscosity increase** | **Gel Structure** |
|---|---|---|---|---|
| Xanthan gum | Xanthan gum | ∼0.05% | Not solubilized | - |
| Silica | Aerosil | ≤10% | Yes, no viscosity increase | - |
| | | ∼20 | Yes, viscosity increase | - |
| Povidone/ Copovidone | plasdone K25 | ∼0.5% | Yes, no viscosity increase | - |
| | | ∼2% | Not solubilized | - |
| | Plasdone K90 | ∼0,5% | Not solubilized | - |
| | Plasdone 5630 | 0.5 < % ≤ 5% | Yes, no viscosity increase | - |
| | | ∼10% | Yes, slight viscosity increase | - |
| HPMC | HMPC 3 | ∼0,5% | Not solubilized | - |
| | HMPC E50 | ∼0,5% | Not solubilized | - |
| | Klucel EF | ≤2% | Yes, no viscosity increase | - |
| | (MW 80.000) | 4≤%≤10% | Yes, viscosity increase | No gel-like structure |
| | | ∼15% (Conc. Max tested) | Yes, viscosity increase | No gel-like structure |
| | Klucel LF | <2% | Yes, no viscosity increase | - |
| | (MW 95.000) | 2≤%≤7% | Yes, viscosity increase | No gel-like structure |
| | | ≥7.5% | Yes, viscosity increase | Gel-like structure |
| HPC | | ∼10% (Conc Max tested) | Yes, viscosity increase | Gel-like structure |
| | Klucel GF | ∼0.5% | Yes, no viscosity increase | - |
| | (MW 370.000) | ∼2% | Yes, slight viscosity increase | No gel-like structure |
| | | ≥2.5% | Yes, viscosity increase | Gel-like structure |
| | | ∼8% (Conc Max tested) | Yes, viscosity increase | Gel-like structure |
| | Klucel HXF | ≤1% | Yes, no viscosity increase | - |
| | (MW | 2≤%≤2.5% | Yes, viscosity increase | No gel-like structure |
| | 1.1500.000) | ≥3% | Yes, viscostity increase | Gel-like structure |
| | finely ground grades | ∼5% (conc. Max tested) | Yes, viscostity increase | Gel-like structure |

Table 35 illustrates the results of testing a variety of polymers to illustrate optimal solubilization in thyme oil and the formation of an acceptable gel structure.

| **Viscosity Enhancer** | | **% (w/w)** | **Solubilization in thyme oil/viscosity increase** | **Gel Structure** |
|---|---|---|---|---|
| HPC | Klucel EF (MW 80,000) | ∼4% | Yes, viscosity increase | No gel-like structure |
| | Klucel LF (mew 95.000) | ∼4% | Yes, viscosity increase | No gel-like structure |
| | Klucel GF (MW 370.000) | ∼4% | Yes, viscosity increase | Gel-like structure |
| | Klucel HXF (MW 1.150.000) Finely ground grades | ∼3% | Yes, viscosity increase | Gel-like structure |

Other embodiments will be apparent to those skilled in the art from consideration of the specification and practice of the devices and methods disclosed herein. It is intended that the specification and examples be considered as exemplary only, with a true scope being indicated by the following claims,

## Claims

1. A hard or soft capsule comprising a fill formulation comprising a mixture of at least one polymer and at least one fill component wherein the at least one fill component in the absence of the at least one polymer will migrate into or through a capsule shell, wherein said mixture is in the form of a gel-like matrix and the fill component comprises a volatile compound,
and wherein the fill component(s) is present in an amount of from 80% to 99.9% w/w,wherein the at least one polymer is hydroxypropyl cellulose, and wherein the at least one fill component is selected from the group consisting of 2-butanol, 2-pentanone, L- carvone, and mixtures thereof.

2. The capsule of claim 1, wherein the capsule is sealed or banded.

3. The capsule of any of the preceding claims, wherein the capsule by weight is selected from the group consisting of cellulose ethers, gelatin, and mixtures thereof, preferably wherein the primary component of the capsule by weight comprises gelatin, or is hydroxypropyl methylcellulose, or is pullulan.

4. A capsule according to any of the preceding claims arranged within an outer capsule such to form an inner and outer capsule and the outer capsule comprises at least one component selected from the group consisting of a therapeutic drug, a pharmaceutical dosage form, a placebo formulation, and combinations thereof.

5. A capsule according to any of the preceding claims enclosing therein an inner capsule such to form an inner and outer capsule wherein the inner capsule comprises a component selected from the group consisting of a therapeutic drug, a placebo formulation, and combinations thereof, and the outer capsule comprises the inner capsule and the fill formulation.

6. The capsule according to anyone of claims 4 or 5, wherein the inner and outer capsule each has a composition and the inner and outer capsule compositions are substantially the same or different.

7. A capsule according to any of the preceding claims wherein the hydroxypropyl cellulose is present in an amount ranging from 0.1% to 20% w/w.

8. Use of a capsule according to any of the preceding claims for preventing and/or retarding migration of the fill component into or through the capsule shell, wherein the gel-like matrix is formed prior to its loading into the capsule shell.

## Patentansprüche

1. Harte oder weiche Kapsel umfassend eine Füllformulierung, die ein Gemisch von wenigstens einem Polymer und wenigstens einer Füllkomponente umfasst, wobei die wenigstens eine Füllkomponente ohne Vorhandensein des wenigstens einen Polymers in oder durch eine Kapselhülle wandern wird, wobei das Gemisch in der Form einer Gel-artigen Matrix vorliegt und die Füllkomponente eine flüchtige Verbindung umfasst,
und wobei die Füllkomponente(n) in einer Menge von 80 % bis 99,9 % w/w vorhanden ist/sind, wobei das wenigstens eine Polymer Hydroxypropylcellulose ist und wobei die wenigstens eine Füllkomponente ausgewählt ist aus der Gruppe bestehend aus 2-Butanol, 2-Pentanon, L-Carvon und Gemischen davon.

2. Kapsel gemäß Anspruch 1, wobei die Kapsel verschlossen oder verbunden ist.

3. Kapsel gemäß einem der vorstehenden Ansprüche, wobei die Kapsel hach Gewicht ausgewählt ist aus der Gruppe bestehend aus Celluloseethern, Gelatine und Gemischen davon, vorzugsweise wobei die Hauptkomponente der Kapsel nach Gewicht Gelatine umfasst oder Hydroxypropylmethylcellulose ist oder Pullulan ist.

4. Kapsel gemäß einem der vorstehenden Ansprüche, angeordnet innerhalb einer Außenkapsel, um eine Innen- und Außenkapsel zu bilden, und wobei die Außenkapsel wenigstens eine Komponente ausgewählt aus der Gruppe bestehend aus einem therapeutischen Arzneimittel, einer pharmazeutischen Darreichungsform, einer Placeboformulierung und Kombinationen davon umfasst.

5. Kapsel gemäß einem der vorstehenden Ansprüche, die darin eine Innenkapsel umschließt, um eine Innen- und Außenkapsel zu bilden, wobei die Innenkapsel eine Komponente ausgewählt aus der Gruppe bestehend aus einem therapeutischen Arzneimittel, einer Placeboformulierung und Kombinationen davon umfasst und die Außenkapsel die Innenkapsel und die Füllformulierung umfasst.

6. Kapsel gemäß einem der Ansprüche 4 oder 5, wobei die Innen- und die Außenkapsel jeweils eine Zusammensetzung aufweisen und die Innen- und Außenkapsel-Zusammensetzungen im Wesentliche gleich oder verschieden sind.

7. Kapsel gemäß einem der vorstehenden Ansprüche, wobei die Hydroxypropylcellulose in einer Menge in dem Bereich von 0,1 % bis 20 % w/w vorhanden ist.

8. Verwendung einer Kapsel gemäß einem der vorstehenden Ansprüche zum Verhindern und/oder Verzögern von Wanderung der Füllkomponente in oder durch die Kapselhülle, wobei die Gel-artige Matrix gebildet wird, bevor sie in die Kapselhülle gefüllt wird.

## Revendications

1. Capsule dure ou molle comprenant une formule de remplissage comprenant un mélange d'au moins un polymère et d'au moins un composant de remplissage, où l'au moins un composant de remplissage en l'absence de l'au moins un polymère migre dans ou au travers d'une enveloppe de capsule, où ledit mélange se présente sous la forme d'une matrice de type gel et le composant de remplissage comprend un composé volatil,
et où le ou les composants de remplissage sont présents à une teneur comprise entre 80 % et 99,9 % en masse, où l'au moins un polymère est l'hydroxypropylcellulose, et où l'au moins un composant de remplissage est choisi dans le groupe constitué par le 2-butanol, la 2-pentanone, la L-carvone et leurs mélanges.

2. Capsule selon la revendication 1, où la capsule est scellée ou enroulée de bandes.

3. Capsule selon l'une quelconque des revendications précédentes, où la capsule en masse est choisie dans le groupe constitué par les éthers de cellulose, la gélatine et leurs mélanges, préférentiellement où le composant principal de la capsule en masse est constitué de gélatine, ou est l'hydroxypropylméthylcellulose, ou est le pullulane.

4. Capsule selon l'une quelconque des revendications précédentes, disposé à l'intérieur d'une capsule extérieure de sorte à former une capsule intérieure et extérieure, et la capsule extérieure comprend au moins un composant choisi dans le groupe constitué par un médicament thérapeutique, une forme galénique, une formule placebo, et leurs combinaisons.

5. Capsule selon l'une quelconque des revendications précédentes, renfermant en son sein une capsule intérieure de sorte à former une capsule intérieure ou extérieure, où la capsule intérieure comprend un composant choisi dans le groupe constitué par un médicament thérapeutique, une formule placebo et leurs combinaisons, et la capsule extérieure comprend la capsule intérieure et la formule de remplissage.

6. Capsule selon l'une quelconque des revendications 4 ou 5, où chacune des capsules intérieure et extérieure présente une composition et les compositions des capsules intérieure et extérieure sont essentiellement identiques ou différentes.

7. Capsule selon l'une quelconque des revendications précédentes, où l'hydroxypropylcellulose est présente à une teneur comprise entre 0,1 % et 20 % en masse.

8. Utilisation d'une capsule selon l'une quelconque des revendications précédentes dans la prévention et/ou le retard de la migration du composant de remplissage dans ou à travers l'enveloppe de la capsule, où la matrice de type gel est formée avant son chargement dans l'enveloppe de la capsule.
